Europäisches Patentamt

European Patent Office

Office européen des brevets

(19)

(11) Veröffentlichungsnummer: **0 207 471 B1**

# EUROPÄISCHE PATENTSCHRIFT

(45) Veröffentlichungstag der Patentschrift: **11.12.91**

(51) Int. Cl.⁵: **G08B 21/00, G01N 33/36**

(21) Anmeldenummer: **86108808.6**

(22) Anmeldetag: **28.06.86**

(54) **Verfahren zur Überwachung der Fadenqualität des laufenden Fadens.**

(30) Priorität: **03.07.85 DE 3523710**

(43) Veröffentlichungstag der Anmeldung:
**07.01.87 Patentblatt 87/02**

(45) Bekanntmachung des Hinweises auf die
Patenterteilung:
**11.12.91 Patentblatt 91/50**

(84) Benannte Vertragsstaaten:
**CH DE FR GB IT LI**

(56) Entgegenhaltungen:
**DE-A- 1 116 921**
**FR-A- 2 548 643**
**GB-A- 681 477**
**US-A- 3 726 137**
**US-A- 4 182 167**

(73) Patentinhaber: **BARMAG AG**
**Leverkuser Strasse 65 Postfach 11 02 40**
**W-5630 Remscheid 11(DE)**

(72) Erfinder: **Martens, Gerhard**
**Höhenweg 71**
**W-5630 Remscheid 11(DE)**

(74) Vertreter: **Pfingsten, Dieter, Dipl.-Ing.**
**Barmag AG Leverkuser Strasse 65 Postfach**
**110240**
**W-5630 Remscheid 11(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Überwachung der Fadenzugkraft des laufenden Fadens, insbesondere zwischen dem Falschdraller und dem Ausgangslieferwerk der Texturierzone einer vielstelligen Falschzwirnkräuselmaschine.

Bei dem Verfahren nach der DE-C 30 05 746 werden in einer vielstelligen Textilmaschine die Meßdaten, die an einer Vielzahl von Meßstellen laufend anfallen, von einer zentralen Datenverarbeitungsanlage erfaßt und bearbeitet. Die Abfragegeschwindigkeit wird dadurch erhöht, daß zwischen der zentralen Datenverarbeitungsanlage und der Vielzahl der Meßstellen mehrere dezentrale Datenverarbeitungseinrichtungen vorgesehen sind. Diesen dezentralen Datenverarbeitungseinrichtungen ist jeweils nur eine begrenzte Anzahl von Meßstellen zugeordnet. Die dezentralen Datenverarbeitungseinrichtungen übernehmen die Abfrage (Scanner) und die Zwischenspeicherungen der Daten.

Dies ist zum einen eine aufwendige Lösung. Zum anderen wird bei dieser Lösung zwar die Abfragegeschwindigkeit und die Abfragefrequenz erhöht. Es bleibt jedoch der Nachteil, daß lediglich die Augenblickswerte der Messung zu den Abfragezeitpunkten erfaßt werden. Dadurch werden nur Zufallswerte ermittelt und ausgewertet, die für den Prozeßverlauf und die erzielte Produktqualität keine zuverlässige Aussage ermöglichen.

Die Erfindung löst die Aufgabe, beim Falschzwirntexturieren in einer Vielzahl von Bearbeitungsstellen mit gleichartigen Meßwerten ein Verfahren anzugeben, das eine zeitlich lückenlose Auswertung der Meßsignale an einer dezentralen Stelle, vorzugsweise an jeder Meßstelle, erlaubt und eine Aussage über die Qualität des zu erzeugenden Fadens macht. Dieses Verfahren ist gekennzeichnet durch die laufende Messung der Fadenzugkraft, die laufende Bildung des Mittelwertes aus den Meßsignalen, den ständigen Vergleich der Meßsignale mit dem laufenden Mittelwert und die Bildung eines Differenzsignales und durch die Auslösung eines Alarmsignals, wenn der Mittelwert oder das Differenzsignal die jeweils vorgegebenen Toleranzbereiche verlassen.

Die Toleranzbereiche werden jeweils identisch für eine Gruppe von Meßstellen, vorzugsweise identisch für sämtliche Meßstellen einer Maschine vorgegeben.

Durch die GB-A 681.477 ist zwar ein Verfahren bekannt, bei dem die Masse eines Spinnkabels (sliver), ihr Mittelwert und die mittlere Abweichung des aktuell gemessenen Massewerts von dem Mittelwert gemessen wird. Hierbei ergeben die Masseverteilung und die daraus abgeleiteten statistischen Werte, also Mittelwert und mittlere Abweichung, eine zusätzliche Aussage über die Masseverteilung. Im Falle der Erfindung ist die Fadenspannung ein Prozeßparameter des Kräuselprozesses. Jedoch können weder die aus der Fadenspannung abgeleitete Mittelwertbildung noch die daraus abgeleitete Differenzbildung zwischen laufender Fadenspannung und Mittelwert der Fadenspannung zur Überwachung des Texturierprozesses, sondern nur zur Bestimmung der Kräuselqualität des bei der Falschzwirntexturierung erzeugten Fadens dienen.

Das angegebene Verfahren erlaubt es, in Falschzwirnkräuselmaschinen die Fadenzugkraft eines jeden Fadens - in modernen Texturiermaschinen laufen z. B. 216 Fäden - laufend zu messen und derart zu erfassen und auszuwerten, daß eine lückenlose Erfassung sämtlicher kritischen Betriebszustände möglich ist. Hierzu wird jeder Fadenzugkraftmesser derart konstruiert und elektrisch geschaltet, daß zur Qualitätsüberwachung zwei maßgebende Signale erzeugt werden, nämlich ein Alarmsignal bei Abweichung des Mittelwerts von einem Toleranzfeld und ein Alarmsignal bei Abweichung der Differenz zwischen Meßsignal und Mittelwert von einem Toleranzfeld. Es hat sich herausgestellt, daß der Mittelwert und die Abweichung des Meßsignals vom Mittelwert hinreichende Aussagekraft für die Qualität des erzeugten Fadens haben.

Die erzeugten Alarmsignale können verwandt werden, um den kritischen Betriebszustand einer Texturierstelle lediglich zu signalisieren oder um diese Texturierstelle - z. B. durch Betätigung des Fadenschneiders - außer Betrieb zu setzen.

Die erzeugten Alarmsignale werden vorzugsweise jeweils für sich ausgewertet zur Auslösung einer der Meßstelle zugeordneten optischen oder akustischen Fehleranzeige oder zur Stillsetzung des Fadenlaufs an der Meßstelle.

Im folgenden wird die Erfindung anhand von Diagrammen und eines vereinfachten Schaltplans beschrieben.

Das Diagramm I zeigt den Ausschnitt eines Fadenzugkraft-Schriebes. Die Abszisse enthält die Zeit. Die Fadenzugkraft ist mit U bezeichnet.

Das Diagramm II zeigt den Mittelwert MU, der sich aus dem Fadenzugkraft-Schrieb nach Diagramm I ergibt. Praktisch wird diese Mittelwertbildung dadurch bewerkstelligt, daß der Meßwert U der Fadenzugkraft einem Filter mit relativ großer Zeitkonstante aufgegeben wird. Durch die Vorwahl der Zeitkonstante kann die Mittelwertbildung beeinflußt werden. Je größer die Zeitkonstante gewählt wird, desto weniger gehen kurzzeitige Schwankungen des Meßsignals in den Mittelwert MU ein.

In Abhängigkeit von dem gebildeten Mittelwert wird ein Alarmsignal A laut Balkendiagramm III erzeugt, wenn der gebildete Mittelwert ein vorgegebenes Toleranzfeld zwischen dem oberen Grenzwert GOMU und dem unteren Grenzwert GUMU verläßt. Durch die Mittelwertbildung läßt sich mithin das langfristige Niveau der Fadenzugkraft erfassen.

Ferner wird laut Diagramm IV aus dem Meßsignal U und dem Mittelwert MU ein Differenzsignal DU erzeugt. Dieses Differenzsignal zeigt an, ob die Fadenzugkraft sich im Rahmen eines Toleranzfeldes um den Mittelwert hält oder ob unzulässige Spannungsschwankungen auftreten. Es zeigt sich anhand der Diagramme, daß allmählich eintretende Änderungen der Fadenzugkraft erst signalisiert werden, wenn das Toleranzfeld zwischen GOMU und GUMU verlassen wird. Die kurzfristigen Schwankungen, selbst wenn sie den Mittelwert nicht nennenswert beeinflussen, werden dagegen über die Differenzbildung DU erfaßt und - wie das Balkendiagramm V zeigt - als Alarmsignal signalisiert, wenn das Differenzsignal DU ein vorgegebenes Toleranzfeld zwischen dem oberen Grenzwert GODU und dem unteren Grenzwert GUDU verläßt.

Es sei erwähnt, daß die Toleranzfelder für den Mittelwert und für den Differenzwert durch Versuche ermittelt werden müssen, nachdem zuvor die gewünschte Fadenqualität definiert worden ist.

Der vereinfachte Schaltplan bezieht sich auf eine Bearbeitungsstelle einer Falschzwirnkräuselmaschine.

Der von einer nicht dargestellten Fadenlieferstelle, z.B. Spule, kommende Faden 40 läuft über Fadenschneider 42 und wird von der Galette 41 abgezogen. Danach läuft der Faden mit Berührung über eine Heizung 43 und durchläuft sodann einen Falschdraller 44 und einen Fadenfühler 1. Sodann wird der Faden von Galette 45 aus der Falschdrallzone abgezogen und mittels einer schematisch dargestellten Aufwickelvorrichtung auf der Spule 46 aufgewickelt.

Als Fadenschneider 42 wird z.B. der im Prospekt "Telemecanique", Oktober 1979, vorgestellte Fadenschneider CA9 verwendet. Der Falschdraller entspricht z.B. dem in den amerikanischen Patenten 3,813,868 (Bag. 838) oder 4,339,915 (Bag. 1187) beschriebenen Falschdraller.

Der Fadenfühler 1 entspricht z.B. dem in der europäischen Patentanmeldung Nr. 86102312.5 dargestellten Fadenfühler.

Der Fadenfühler erzeugt ein von der Fadenspannung des Fadens 40 zwischen Falschdraller 44 und Galette 45 abhängiges Ausgangssignal.

Das Ausgangssignal wird einem von einer strichpunktierten Linie umgebenen Schaltkreis 47 zugeführt. Der Schaltkreis 47 ist jeder Stelle einer mehrstelligen Falschdrallvorrichtung und jedem Fadenfühler 1 einer jeden Stelle zugeordnet. Der Schaltkreis 47 erhält von einem weiter unten näher beschriebenen Grenzwert-Speicher 48 vorgegebene Grenzwerte. Der Speicher 48 ist einer Gruppe von Texturierstellen einer mehrstelligen Texturiervorrichtung zugeordnet. Der Schaltkreis 47 erzeugt ein Ausgangssignal, das dem Fadenschneider 42 zugeführt wird, und ein weiteres Ausgangssignal, das einer Hauptalarmeinrichtung 49 zugeführt wird, die ebenfalls einer Gruppe von Texturierstellen zugeordnet ist. Ferner erzeugt der Schaltkreis 47 Ausgangssignale, die den weiter unten beschriebenen Alarmeinrichtungen 50, 51, 52, 53 zugeführt werden. Diese Alarmeinrichtungen sind allen Texturierstellen zugeordnet.

Das vom Fadenfühler 1 erzeugte Ausgangssignal wird dem Verstärker 54 und danach dem Filter 55 zugeführt. Der Filter 55 besteht aus einer Induktionsspule und einem Kondensator und hat eine Verzögerungszeitkonstante von 1 bis 3 Sekunden. Das Ausgangssignal des Verstärkers 54 ist eine Spannung U, die über Leitung 56 einem zentralen Mikroprozessor zur weiteren Verarbeitung und Auswertung zugeführt wird. Das Ausgangssignal des Filters 55 ist der Mittelwert MU, der über Leitung 57 ebenfalls einem zentralen Mikroprozessor zur weiteren Verarbeitung und Auswertung zugeführt werden kann. Außerdem werden Eingangssignale U und MU dem Differentialverstärker 58 zugeführt, der ein die Differenz der Eingangssignale U und MU darstellendes Ausgangssignal DU erzeugt. Das Ausgangssignal DU des Differentialverstärkers 58 kann über Leitung 59 dem zentralen Mikroprozessor zur weiteren Verarbeitung und Auswertung zugeführt werden.

Das Ausgangssignal MU des Filters 55 dient weiterhin zur Auslösung von Alarmsignalen A1 and A2, wenn der Mittelwert MU das vorgegebene Toleranzfeld verläßt. Das vorgegebene Toleranzfeld liegt zwischen einem oberen Grenzwert des Mittelwerts GOMU und einem unteren Grenzwert des Mittelwerts GUMU. Beide Grenzwerte werden in dem Grenzwert-Speicher 48 gespeichert und über entsprechende Leitungen dem Schaltkreis 47 zugeführt. Zu diesem Zweck sind in dem Schaltkreis 48 Trigger 60 und 61 vorgesehen. Der Trigger 60 erhält den Mittelwert MU und den oberen Grenzwert des Mittelwerts GOMU und erzeugt ein Ausgangssignal A1, sobald der Mittelwert den vorgegebenen oberen Grenzwert des Mittelwerts übersteigt. Der Trigger 61 erhält als Eingangssignal den Mittelwert MU und den vorgegebenen unteren Grenzwert des Mittelwerts GUMU und erzeugt ein Ausgangssignal A2, wenn der Mittelwert MU unter dem vorgegebenen unteren Grenzwert des Mittelwertes liegt.

Ferner erzeugt der Schaltkreis 47 Alarmsignale A3, A4, wenn das Differenzsignal DU die vorgegebenen Toleranzbereiche zwischen einem vorgegebenen oberen Grenzwert des Differenzwerts GODU und einem vorgegebenen unteren Grenzwert des Differenzwerts GUDU verläßt. Der obere und der untere Grenzwert werden in dem Grenzwert-Speicher 48 gespeichert und als Eingangssignale den Triggern 62, 63 des Schaltkreises 47 zugeführt, wobei Trigger 62 den oberen und Trigger 63 den unteren Grenzwert erhält. Als weiteres Eingangssignal wird den Triggern 62 und 63 das Ausgangssignal des bereits erwähnten Differentialverstärkers 58, nämlich das Differenzsignal DU, zugeführt. Übersteigt das Differenzsignal DU den vorgegebenen oberen Grenzwert GODU, so erzeugt der Trigger 62 ein Alarmsignal A3. Sinkt das Differenzsignal DU unter den vorgegebenen unteren Grenzwert GUDU, so erzeugt der Trigger 63 ein Alarmsignal A4. Die Alarmsignale A1, A2, A3, A4 werden jeweils einer der Alarmeinrichtungen 50 bis 53 zugeführt, welche der Texturierstelle zugeordnet und beispielsweise als in den Schaltkreis 47 integrierte Leuchtdioden ausgebildet sind. Außerdem werden die Alarmsignale A1 bis A4 dem ODER-Glied 64, der Verzögerungsstufe 65, dem Speicher 66 und dem Verstärker 67 zugeführt. Das ODER-Glied 64 erzeugt ein Ausgangssignal, sobald eines der Alarmsignale A1 bis A4 anliegt. Die Verzögerungsstufe besitzt eine Verzögerungszeitkonstante von etwa 10 ms und verhindert, daß durch eine kurzfristige und unerhebliche Störung des Texturierbetriebes ein Ausgangssignal erzeugt wird, das dazu führt, daß der Fadenschneider 42 den Faden 40 abschneidet. Durch den Speicher 66 wird gewährleistet, daß eine Hauptalarmeinrichtung 49, die einer Gruppe von Stellen oder der gesamten Maschine zugeordnet ist, ein Dauersignal erzeugt, das die Störung und/oder Unterbrechung des Betriebes anzeigt.

Das Ausgangssignal des Speichers 66 wird außerdem einem Verstärker 67 zugeführt. Von dort gelangt das Ausgangssignal zusammen mit einem anderen Signal, auf das später näher eingegangen wird, zum ODER-Glied 68. Auf das Ausgangssignal des Verstärkers 67 hin erzeugt das ODER-Glied 68 ein Ausgangssignal, das dem Fadenschneider 42 zugeführt wird und diesen veranlaßt, den Faden abzuschneiden und somit den Texturier- bzw. Strecktexturierprozeß zu unterbrechen. Der Trigger 69 führt dem ODER-Glied 68 über Verzögerungsstufe 70 und Verstärker 71 ein weiteres Eingangssignal zu. Der Trigger 69 erhält das die gemessene Fadenzugkraft darstellende Signal U sowie einen zweiten vorgegebenen Wert LLU, der in dem Grenzwert-Speicher 48 gespeichert wird und den niedrigsten zugelassenen Wert der Fadenzugkraft darstellt. Es sei erwähnt, daß dieser Wert LLU vorzugsweise Null beträgt. Der Trigger 69 erzeugt ein Ausgangssignal, wenn der gemessene Wert U niedriger oder gleich dem vorgegebenen Wert LLU ist. Die Verzögerungszeitkonstante der Verzögerungsstufe 70 beträgt etwa 10 ms. Wenn die Fadenzugkraft unter einen vorgegebenen Wert sinkt bzw. ein Fadenbruch zwischen den Galetten 41 und 45 vorliegt, gibt der Trigger 69 - wie oben bereits erwähnt - an das ODER-Glied 68 ein Ausgangssignal ab, welches bewirkt, daß der Fadenschneider 42 den Faden 40 oberhalb der Galette 41 abschneidet.

BEZUGSZEICHENAUFSTELLUNG

| 40 | Faden |
| 41 | Galette |
| 42 | Fadenschneider |
| 43 | Heizung |
| 44 | Falschdraller |
| 45 | Galette |
| 46 | Spule |
| 47 | Stromkreis |
| 48 | Speicher |
| 49 | Hauptalarmeinrichtung |
| 50 ) | |
| 51 ) | Alarmeinrichtung |
| 52 ) | |
| 53 ) | |
| 54 | Verstärker |
| 55 | Filter |
| 56 | Leitung |
| 57 | Leitung |
| 58 | Differentialverstärker |
| 59 | Leitung |
| 60 ) | |
| 61 ) | Trigger |
| 62 ) | |
| 63 ) | |
| 64 | ODER-Glied |
| 65 | Verzögerungsstufe |
| 66 | Speicher |
| 67 | Verstärker |
| 68 | ODER-Glied |
| 69 | Trigger |
| 70 | Verzögerungsstufe |
| 71 | Verstärker |

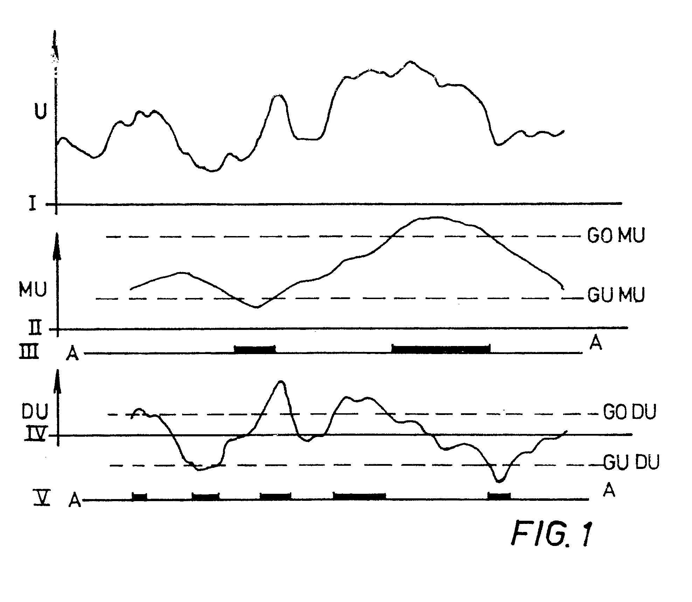

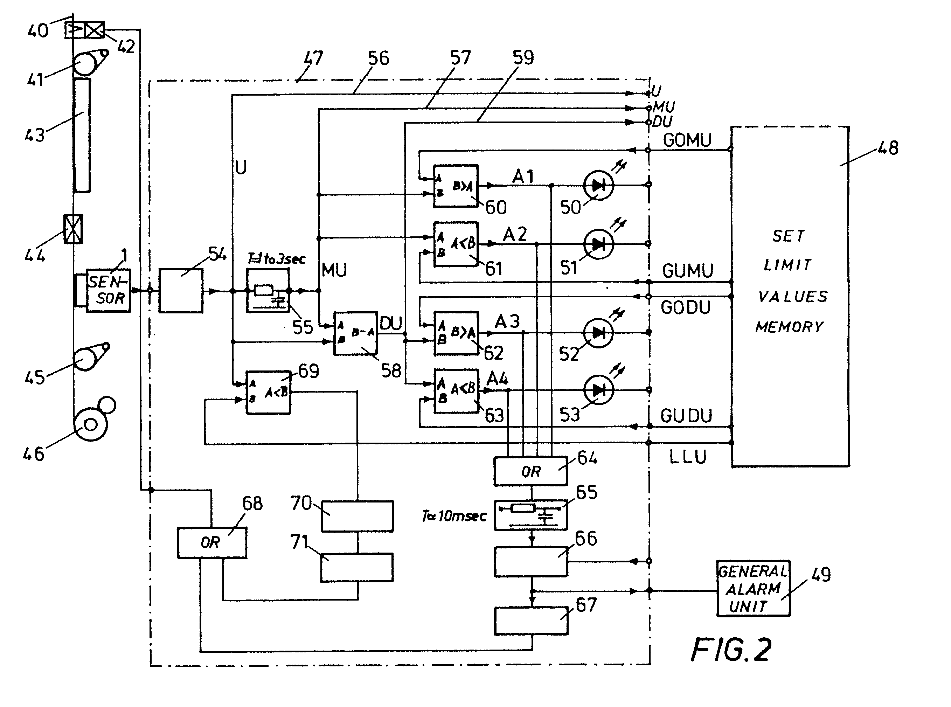

**Patentansprüche**

1. Verfahren zur laufenden Überwachung der Fadenqualität eines durch Falschdralltexturierung gekräuselten Fadens durch Messung der Fadenzugkraft des laufenden Fadens zwischen dem Falschdraller und dem Ausgangslieferwerk der Texturierzone einer vielstelligen Falschzwirnkräuselmaschine,
gekennzeichnet durch die folgenden Merkmale:
Die Fadenzugkraft wird laufend gemessen (Meßsignal: U); die laufenden Meßwerte (U) werden an der Meßstelle zu einem Mittelwert (MU) laufend zusammengefaßt;
aus dem laufenden Mittelwert (MU) und dem laufenden Meßwert wird an der Meßstelle das Differenzsignal (DU) laufend gebildet;

ein Alarmsignal (A) wird an der Meßstelle erzeugt, wenn der Mittelwert (MU) ein vorgegebenes Toleranzfeld zwischen einem oberen Grenzwert des Mittelwerts (GOMU) und einem unteren Grenzwert des Mittelwerts (GUMU) verläßt und/oder wenn das laufend gebildete Differenzsignal (DU) ein zweites vorgegebenes Toleranzfeld zwischen einem oberen Grenzwert des Differenzwertes (GODU) und einem unteren Grenzwert des Differenzwertes (GUDU) verläßt.

## Claims

1. Method for continuously monitoring the quality of a yarn which is crimped by false-twist crimping by measuring the tensile strength of the running yarn between the false twister and the delivery device of the texturing zone of a multistation false-twist crimping machine, characterised by the following features:

   The yarn tensile strength is continuously measured (measuring signal: U);

   the current measured values (U) are continuously collected at the measuring point to form a mean value (MU);

   the differential signal (DU) is continuously formed at the measuring point from the current mean value (MU) and the current measured value;

   an alarm signal (A) is produced at the measuring point if the mean value (MU) departs from a predetermined tolerance zone between an upper limit value (GOMU) of the mean value and a lower limit value (GUMU) of the mean value and/or if the continuously formed differential signal (DU) departs from a second predetermined tolerance zone between an upper limit value (GODU) of the differential value and a lower limit value (GUDU) of the differential value.

## Revendications

1. Procédé pour la surveillance en continu de la qualité d'un fil frisé par texturation à fausse-torsion, par mesure de la tension du fil en défilement entre le dispositif à fausse-torsion et le dispositif de sortie de la zone de texturation d'une machine à friser les filés à fausse-torsion à plusieurs points de traitement, caractérisé par les caractéristiques suivantes :

   la tension du fil est mesurée en continu (signal de mesure : U) ;

   les valeurs de mesure courantes (U) sont composées au point de mesure pour former une valeur moyenne (MU) ;

   le signal différentiel (DU) au point de mesure est formé en continu à partir de la valeur moyenne courante (MU) et de la valeur de mesure courante ;

   un signal d'avertissement (A) est produit au point de mesure lorsque la valeur moyenne (MU) sort d'une plage de tolérances prédéterminée comprise entre un seuil supérieur de la valeur moyenne (GOMU) et un seuil inférieur de la valeur moyenne (GUMU) et/ou lorsque le signal diférentiel (DU) formé en continu sort d'une deuxième plage de tolérances prédéterminée comprise entre un seuil supérieur de la valeur différentielle (GODU) et un seuil inférieur de la valeur différentielle (GUDU).

*FIG. 1*

FIG.2

EP 0 207 471 B1